(19) Europäisches Patentamt
European Patent Office
Office européen des brevets

(11) Veröffentlichungsnummer: **0 063 767**
**B1**

(12) **EUROPÄISCHE PATENTSCHRIFT**

(45) Veröffentlichungstag der Patentschrift: **12.09.84**

(51) Int. Cl.³: **C 07 C 143/74**

(21) Anmeldenummer: **82103244.8**

(22) Anmeldetag: **17.04.82**

# (54) 2-Ketosulfonamide und Verfahren zu ihrer Herstellung.

(30) Priorität: **23.04.81 DE 3116129**

(43) Veröffentlichungstag der Anmeldung: **03.11.82 Patentblatt 82/44**

(45) Bekanntmachung des Hinweises auf die Patenterteilung: **12.09.84 Patentblatt 84/37**

(84) Benannte Vertragsstaaten: **BE CH DE FR GB IT LI NL**

(56) Entgegenhaltungen:
**HELVETICA CHIMICA ACTA, Band 45, Nr. 84-85, 1962, Seiten 717-728, Basel, CH. B.J.R. NICOLAUS et al.: "Auf das Zentralnervensystem wirkende Substanzen XXIX. Über neuartige schwefelhaltige Heterocyclen: 4,4-Dialkyl-1,2-thioazetidin-3-on-1,1-dioxide und 2,4,4-Trialkyl-1,2-thioazetidin-3-on1,1-dioxide"**

(73) Patentinhaber: **HOECHST AKTIENGESELLSCHAFT, Postfach 80 03 20, D-6230 Frankfurt am Main 80 (DE)**

(72) Erfinder: **Günther, Dieter, Dr., Nachtigallenweg 1A, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der vorliegenden Erfindung sind neue 2-Ketosulfonamide der allgemeinen Formel I

$$R-\overset{O}{\overset{\|}{C}}-CH_2-SO_2NH_2 \qquad (I)$$

in der R einen Alkylrest mit 1 bis 4 Kohlenstoffatomen bedeutet, und ein Verfahren zu ihrer Herstellung, das dadurch gekennzeichnet ist, dass man Verbindungen der allgemeinen Formel II

$$R-CHOH-CH_2-SO_2NH_2 \qquad (II)$$

in der R die obengenannte Bedeutung besitzt, in einem leicht flüchtigen, oxidationsbeständigen organischen Lösungsmittel bei einer Temperatur zwischen 0°C und dem Siedepunkt des Lösungsmittels mit Mangandioxid oxidiert.

Bevorzugt verwendet man Aceton und Acetonitril als Lösungsmittel.

Die Temperatur ist vorzugsweise 20 bis 40°C.

Im allgemeinen wird die Oxidation mit der 4- bis 12fachen Gewichtsmenge Mangandioxid, bezogen auf die Menge der eingesetzten Verbindung II, durchgeführt. Nach Beendigung der Umsetzung kann das Mangandioxid durch eine einfache Filtration abgetrennt und das Lösungsmittel im Vakuum abdestilliert werden. Man erhält so die kristalline Ketoverbindung, die, falls erforderlich, umkristallisiert werden kann. Die Ausgangsverbindungen der allgemeinen Formel II können durch Umsetzung der entsprechenden Sulfonylchloride $R-CHOH-CH_2SO_2Cl$ mit Ammoniak im Autoklaven bei 100°C hergestellt werden.

Die erfindungsgemässen 2-Ketosulfonamide der Formel I, insbesondere wenn R für die Methylgruppe steht, stellen Analoga der Acylessigsäureamide dar, die ihrerseits grosse Bedeutung und Interesse als reaktionsfähige Zwischenprodukte besitzen. Die neuen 2-Ketosulfonamide stellen somit wertvolle Zwischenprodukte dar, die es beispielsweise gestatten, aufgrund der reaktionsfähigen Carbonyl- und Methylengruppen aliphatische Sulfonamidgruppen in Zielmoleküle einzuführen. Weiter können sie beispielsweise als Kupplungskomponenten für Azofarbstoffe und -pigmente Verwendung finden. Der folgende Versuchsbericht betrifft die Herstellung eines Azopigments aus dem erfindungsgemässen Acetonsulfonamid und 2-Chlor-4-nitranilin.

*Versuchsbericht:*

8,6 g (50 mMol) 2-Chlor-4-nitranilin werden in einer Mischung von 12 ml konz. Salzsäure und 12 ml Wasser gelöst und auf 0°C abgekühlt. Die Lösung wird mit 14 ml (53,5 mMol) einer 20%igen Natriumnitritlösung versetzt und 15 Min bei 0°C nachgerührt. Danach wird das überschüssige Natriumnitrit mit Amidosulfonsäure zerstört.

Die Diazoniumlösung wird bei 5°C in eine Lösung von 7 g (50 mMol) Acetonsulfonamid in 50 ml Wasser getropft, und dabei wird der pH-Wert zwischen 3 und 5 gehalten. Nach 4 Stunden wird der ausgefallene Farbstoff abgesaugt und getrocknet. Man erhält 11 g eines gelben Pigments der Formel

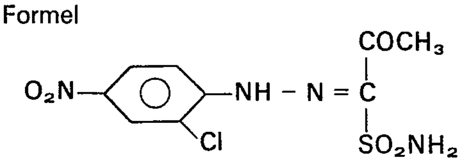

Der besondere Vorteil dieser Pigmente gegenüber Acetoacetamidanalogon ist die Möglichkeit, durch Verlacken der Sulfonamidgruppe mit Salzen z.B. des Bariums, Calciums oder Aluminiums, die Löslichkeit des Pigments noch weiter herabzusetzen.

Die folgenden Beispiele sollen die Erfindung erläutern ohne sie einzuschränken.

*Beispiel 1*

70 g (0,5 Mol) 2-Hydroxypropansulfonamid werden in 700 ml Aceton 4 Stunden mit 600 g Mangandioxid bei Siedetemperatur gerührt. Man saugt vom Rückstand ab und destilliert das Aceton ab. Nach Umkristallisieren des Rohproduktes aus 65 ml i-Propanol erhält man 39,6 g (57,8% d.Th.) an Acetonsulfonamid vom Schmelzpunkt 73 bis 75°C.

Die Mutterlauge der Umkristallisation wird vom i-Propanol befreit, und der Rückstand erneut mit 140 g Mangandioxid oxidiert. Man erhält weitere 7,4 g Produkt vom Schmelzpunkt 74 bis 75°C.

Die Gesamtausbeute beträgt damit 68% d.Th.

*Analyse* für $C_3H_7NO_3S$: (Molekulargewicht 137,16)

Berechnet: C 26,3 H 5,1 N 10,2 O 35,0 S 23,4%

Gefunden: C 26,4 H 4,9 N 10,2 O 34,8 S 22,9%

*Beispiel 2*

82,5 g 2-Hydroxypropansulfonamid werden in 700 ml Aceton zusammen mit 660 g Mangandioxid 6 Stunden bei Raumtemperatur gerührt. Nach Absaugen des Rückstandes und Abdestillieren des Acetons erhält man 80 g Rohprodukt, das aus 70 ml i-Propanol umkristallisiert 61,4 g (75,5% d.Th.) Acetonsulfonamid vom Schmelzpunkt 72 bis 74°C ergibt.

*Beispiel 3*

13,9 g (0,1 Mol) 2-Hydroxypropansulfonamid werden zusammen mit 150 ml Acetonitril und 125 g Mangandioxid 6 Stunden bei Raumtemperatur gerührt. Nach Abtrennen des Rückstands und Abdestillieren des Lösungsmittels erhält man nach Umkristallisieren aus 18 ml i-Propanol 6,65 g (48,5% d.Th.) Acetonsulfonamid vom Schmelzpunkt 70 bis 73°C.

## Patentansprüche

1. 2-Ketonsulfonamide der allgemeinen Formel (I)

$$R-\overset{\overset{O}{\|}}{C}-CH_2-SO_2NH_2 \qquad (I)$$

wobei R ein Alkylrest mit 1 bis 4 Kohlenstoffatomen ist.

2. Verfahren zur Herstellung von 2-Ketosulfonamiden der allgemeinen Formel (I)

$$R-\overset{\overset{O}{\|}}{C}-CH_2-SO_2NH_2 \qquad (I)$$

wobei R ein Alkylrest mit 1 bis 4 Kohlenstoffatomen ist, dadurch gekennzeichnet, dass man Verbindungen der allgemeinen Formel (II)

$$R-CHOH-CH_2-SO_2NH_2 \qquad (II)$$

in der R die oben genannte Bedeutung besitzt, in einem leicht flüchtigen, oxidationsbeständigen organischen Lösungsmittel bei einer Temperatur zwischen 0°C und dem Siedepunkt des Lösungsmittels mit Mangandioxid oxidiert.

3. Verfahren nach Anspruch 2, dadurch gekennzeichnet, dass man als Lösungsmittel Aceton oder Acetonitril verwendet.

4. Verfahren nach den Ansprüchen 1 oder 2, dadurch gekennzeichnet, dass man bei einer Temperatur von 20 bis 40°C arbeitet.

## Claims

1. A 2-ketosulfonamide of the general formula (I)

$$R-\overset{\overset{O}{\|}}{C}-CH_2-SO_2NH_2 \qquad (I)$$

in which R is an alkyl radical having 1 to 4 carbon atoms.

2. A process for the preparation of 2-ketosulfonamides of the general formula (I)

$$R-\overset{\overset{O}{\|}}{C}-CH_2-SO_2NH_2 \qquad (I)$$

in which R is an alkyl radical having 1 to 4 carbon atoms, which process is characterized by oxidizing compounds of the general formula (II)

$$R-CHOH-CH_2SO_2NH_2 \qquad (II)$$

in which R has the above-mentioned meaning, with manganese dioxide in a readily volatile, oxidation-stable organic solvent at a temperature between 0°C and the boiling point of the solvent.

3. The process as claimed in claim 2, wherein the solvent used is acetone or acetonitrile.

4. The process as claimed in claim 1 or 2, wherein a temperature of 20 to 40°C is used.

## Revendications

1. Oxo-2 sulfonamides répondant à la formule générale (I)

$$R-\overset{\overset{O}{\|}}{C}-CH_2-SO_2NH_2 \qquad (I)$$

dans laquelle R représente un radical alkyle contenant de 1 à 4 atomes de carbone.

2. Procédé de préparation d'oxo-2 sulfonamides répondant à la formule générale (I)

$$R-\overset{\overset{O}{\|}}{C}-CH_2-SO_2NH_2 \qquad (I)$$

dans laquelle R représente un radical alkyle contenant de 1 à 4 atomes de carbone, procédé caractérisé en ce qu'on oxyde des composés répondant à la formule générale (II)

$$R-CHOH-CH_2-SO_2NH_2 \qquad (II)$$

dans laquelle R a la signification indiquée ci-dessus, au moyen du dioxyde de manganèse, dans un solvant organique volatile résistant à l'oxydation, à une température comprise entre 0°C et le point d'ébullition du solvant.

3. Procédé selon la revendication 2, caractérisé en ce qu'on utilise, comme solvant, l'acétone ou l'acétonitrile.

4. Procédé selon l'une des revendications 2 ou 3, caractérisé en ce qu'on opère à une température de 20 à 40°C.